# EUROPEAN PATENT SPECIFICATION

(11) **EP 3 511 706 B1**
(45) Date of publication and mention of the grant of the patent: **01.12.2021**
(21) Application number: 17814509.0
(22) Date of filing: 09.05.2017
(51) Int. Cl.: A61N 1/05

(54) **MICROELECTRODE ARRAY AND MANUFACTURING METHOD THEREFOR**
MIKROELEKTRODENANORDNUNG UND HERSTELLUNGSVERFAHREN DAFÜR
RÉSEAU DE MICROÉLECTRODES ET SON PROCÉDÉ DE FABRICATION

(30) Priority: 23.06.2016 CN 201610460562; 28.06.2016 CN 201610486521; 28.09.2016 CN 201610858458; 30.03.2017 CN 201710203187
(43) Date of publication of application: 17.07.2019
(73) Proprietor: Shenzhen Cas-Envision Medical Technology Co., Ltd, Shenzhen, Guangdong 518000 (CN)
(72) Inventor: XIA, Kai, Shenzhen Guangdong 518055 (CN); WU, Tianzhun, Shenzhen Guangdong 518055 (CN); SUN, Bin, Shenzhen Guangdong 518055 (CN); ZENG, Qi, Shenzhen Guangdong 518055 (CN)
(74) Representative: de Arpe Fernandez, Manuel
(86) International application number: PCT/CN2017/083627
(87) International publication number: WO 2017/219771

(56) References cited:
- WO-A1-2010/025547
- CN-A- 102 086 018
- CN-A- 103 196 966
- CN-A- 103 207 225
- CN-A- 106 037 719
- CN-A- 106 108 891
- CN-A- 106 419 906
- US-A1- 2009 061 451
- US-A1- 2014 222 123
- US-B2- 6 974 533
- US-B2- 9 341 590
- C. BOEHLER ET AL: "Nanostructured platinum grass enables superior impedance reduction for neural microelectrodes", BIOMATERIALS, vol. 67, 1 October 2015 (2015-10-01), pages 346-353, XP055695133, AMSTERDAM, NL ISSN: 0142-9612, DOI: 10.1016/j.biomaterials.2015.07.036
- YOUNG-HYUN JIN ET AL: "A novel platinum nanowire-coated neural electrode and its electrochemical and biological characterization", IEEE 24TH INTERNATIONAL CONFERENCE ON MICRO ELECTRO MECHANICAL SYSTEMS (MEMS 2011), IEEE, US, 23 January 2011 (2011-01-23), pages 1003-1006, XP031982584, DOI: 10.1109/MEMSYS.2011.5734597 ISBN: 978-1-4244-9632-7
- JASON K. KAWASAKI ET AL: "Synthesis of Platinum Dendrites and Nanowires Via Directed Electrochemical Nanowire Assembly", NANO LETTERS, vol. 11, no. 2, 9 February 2011 (2011-02-09), pages 781-785, XP055695121, US ISSN: 1530-6984, DOI: 10.1021/nl1039956
- WANG, CUNCHANG et al.: "Glucose Biosensor Based on Platinum Nanowire Array", CHEMICAL SENSORS, vol. 27, no. 3, 30 September 2007 (2007-09-30), pages 29-33, XP009514189, ISSN: 1008-2298, DOI: 10.3969/j.issn.1008-2298.2007.03.005
- FENG, YE et al.: "Pt-Ir02 nanorod array electrode for oxygen evolution in PEM water electrolysis cell", ASIA-PACIFIC JOURNAL OF CHEMICAL ENGINEERING, 3 August 2012 (2012-08-03), pages 271-277, XP055586195, ISSN: 1932-2135, DOI: 10.1002/apj.1675

## Description

### FIELD OF THE INVENTION

The invention relates to the technical field of surface modification of microelectrodes, in particular to a microelectrode array and a method for preparing the same.

### BACKGROUND OF THE INVENTION

As one of the most important implantable micro devices, nerve stimulation/recording electrodes used to stimulate nerve tissue or record nerve electrical signals (electrocardiogram, electroencephalogram, electrocorticogram signals, etc.) are widely used in the field of life sciences, such as brain-computer interface, neurophysiology and brain science research, as an important research and diagnosis tool.

In order to reduce implant trauma while providing more stimulation patterns to the clinic and increasing the resolution of electrical stimulation or recording, nerve stimulation/recording electrodes are moving toward miniaturization and array, i.e. microelectrode array. However, the reduction in electrode size leads to a problem of an increase in electrode impedance and ultimately affects the stimulation efficiency of the electrode. At present, without increasing the physical size of the electrode, the actual surface area of the electrode is increased mainly by surface modification, and thus improving the electrochemical performance of the electrode.

Specifically, the method for surface modification of microelectrode arrays comprises: 1. modifying a layer of platinum black on the surface of the electrode ( Anal. Chem. 1987, 59, 217-218 ); such method reduces the electrochemical impedance of the electrode by at least one order of magnitude, but the electrode exhibits poor mechanical stability; further, the modified layer contains toxic substances such as lead, making it unsuitable for biomedical applications; 2. modifying a layer of platinum gray on the surface of the electrode ( US Patent 6974533, 2005 ); this coating exhibits good mechanical stability and is non-toxic, but its surface roughness is not large enough, which limits its application to surface modification of ultra-high resolution electrodes; 3. modifying microelectrode with iridium oxide ( Eng. Med. Biol. Soc. 2004, 4153-4156 ) or conductive polymers ( J. Biomed. Mater. Res. 2001, 56, 261-272 ); the modified microelectrodes have excellent electrochemical properties (such as high charge injection capacity), but these materials exhibit poor adhesion performance and are likely to fall off the electrode surface during the stimulation process.

In the state of the art is the article: C. BOEHLER ET AL: "Nanostructured platinum grass enables superior impedance reduction for neural microelectrodes".

The publication patent WO 2010/025547A1 discloses a nanostructured microelectrodes and biosensing devices.

US 2014/222123A1 discloses a nanopillar electrode device, comprising a substrate patterned with a plurality of metal pads. The device may further comprise a plurality of nanopillar electrode arrays, wherein each nanopillar electrode array is attached to the substrate above a metal pad and electrically connected to the pad.

### SUMMARY OF THE INVENTION

In view of this, the present invention aims to provide a microelectrode array as defined by independent claim 1 having a modification layer disposed on a surface of a microelectrode thereof. The modification layer adheres well to a substrate of the microelectrode. The microelectrode has a large surface area and a large surface roughness, and is non-toxic.

The modification layer comprises a platinum nanopillar modification layer.

In one embodiment, the platinum nanopillar modification layer, in which the platinum nanopillar may have a diameter of 50 nm to 500 nm, may have a thickness of 500 nm to 5 µm.

In one embodiment, the platinum nanopillar modification layer has a three-dimensional nanoporous structure.

An iridium oxide layer is disposed on the surface of the platinum nanopillar modification layer. The combination of platinum nanopillar modification layer and the iridium oxide layer forms an iridium oxide/platinum nanopillar composite coating. The iridium oxide layer has a thickness of 10 nm to 500nm

In one embodiment, the iridium oxide/platinum nanopillar composite coating has a three-dimensional nanoporous structure, and the porous iridium oxide/platinum nanopillar composite coating has a pore diameter of 50 nm to 500 nm.

In a second aspect, the present invention provides a method defined by independent claim for preparing a microelectrode array, wherein a modification layer is prepared on a surface of a microelectrode to be modified by means of electrodeposition to form a microelectrode array, and wherein the modification layer comprises a platinum nanopillar modification layer.

The method for preparing the microelectrode array comprises:
(1) adding an appropriate amount of a weak reducing agent to a platinum salt solution while mixing to obtain an electrodeposition solution A;
(2) forming a three-electrode system using a platinum plate as a counter electrode, Ag/AgCl as a reference electrode, a microelectrode to be modified as a working electrode, and the electrodeposition solution A, the system connecting to an electrochemical workstation; and
(3) performing electrodeposition at normal temperature and pressure for 300-600s such that a platinum nanopillar modification layer forms on the surface of the microelectrode to obtain a microelectrode array.

The electrodeposition comprises constant potential deposition, constant current deposition or pulse electrodeposition.

The platinum nanopillar modification layer, in which the platinum nanopillar may have a diameter of 50 nm to 500 nm, may have a thickness of 500 nm to 5 µm, and the platinum nanopillar modification layer has a three-dimensional nanoporous structure.

An iridium oxide layer is prepared on the surface of the platinum nanopillar modification layer, the method of which comprises: adding an appropriate amount of an oxidizing agent and weak acid to an iridium salt solution while mixing to obtain an electrodeposition solution B; forming a three-electrode system using a platinum plate as a counter electrode, Ag/AgCl as a reference electrode, the microelectrode array provided with a platinum nanopillar modification layer as a working electrode, and the electrodeposition solution B, the system connecting to an electrochemical workstation;performing electrodeposition at normal temperature and pressure for 300-600s such that an iridium oxide layer forms on the surface of the platinum nanopillar modification layer and a combination of the platinum nanopillar modification layer and the iridium oxide layer forms an iridium oxide/platinum nanopillar composite coating.

Optionally, the iridium salt solution is one or more selected from the group consisting of chloroiridic acid, iridium oxide, iridium chloride, iridium acetylacetonate, sodium hexachloroiridate, potassium hexachloroiridate, ammonium hexachloroiridate, potassium hexanitroiridate and tetrairidium dodecacarbonyl.

Optionally, the oxidizing agent is one or more selected from the group consisting of hydrogen peroxide, oxygen, ozone, potassium peroxide and sodium peroxide; the weak acid is one or more selected from the group consisting of formic acid, acetic acid, oxalic acid and carbonic acid.

Optionally, in the electrodeposition solution B, the concentration of the iridium salt is in a range of 1 mmol/L to 5 mmol/L, the concentration of the oxidizing agent is in a range of 1 mmol/L to 5 mmol/L, and the concentration of the weak acid is in a range of 1 mmol/L to 5 mmol/L.

Optionally, the platinum salt solution is one or more selected from the group consisting of platinum nitrate, platinum chloride, chloroplatinic acid, ammonium hexachloroplatinate, sodium chloroplatinate, potassium hexachloroplatinate and potassium tetrachloroplatinate.

Optionally, the weak reducing agent is one or more selected from the group consisting of formic acid, hydroxylammonium chloride, citric acid, citrate, ascorbic acid, ascorbate, benzene-1,4-diol, benzene-1,2,3-triol and benzene-1,2,4-triol.

Optionally, in the electrodeposition solution A, the concentration of the platinum salt is in a range of 1 mmol/L to 20 mmol/L, and the concentration of the weak reducing agent is in a range of 1 mmol/L to 20 mmol/L.

Some advantages of the embodiments of the present invention will be set forth below, and other advantages will be apparent from the description or the implementation of the embodiments.

### BRIEF DESCRIPTION OF THE DRAWINGS

Fig. 1 shows a scanning electron microscope (SEM) image of a platinum nanopillar modification layer prepared in Example 1 of the present invention.
Fig. 2 shows a comparison between cyclic voltammogram (CV) of the platinum nanopillar modified microelectrode array prepared in Example 1 and an unmodified microelectrode.
Fig. 3 shows a comparison between electrochemical impedance spectrum (EIS) of the platinum nanopillar modified microelectrode array prepared in Example 1 and an unmodified microelectrode.
Fig. 4 shows a scanning electron microscope (SEM) image of an iridium oxide layer prepared in Example 14.
Fig. 5 shows a scanning electron microscope (SEM) image of an iridium oxide/platinum nanopillar composite coating prepared in Example 14.
Fig. 6 shows a comparison between cyclic voltammogram (CV) of an iridium oxide/platinum nanopillar composite coating modified microelectrode array prepared by Example 14, an unmodified microelectrode, and a platinum nanopillar modified microelectrode array.
Fig. 7 shows a comparison between electrochemical impedance spectrum (EIS) of an iridium oxide/platinum nanopillar composite coating modified microelectrode array prepared by Example 14, an unmodified microelectrode, and a platinum nanopillar modified microelectrode array.
Fig. 8 shows a scanning electron microscope (SEM) image of a platinum nanowire modification layer prepared in Example 27 at 10Kx magnification.
Fig. 9 shows a scanning electron microscope (SEM) image of a platinum nanowire modification layer prepared in Example 27 at 15Kx magnification.
Fig. 10 shows a scanning electron microscope (SEM) image of a platinum nanowire modification layer prepared in Example 27 at 20Kx magnification.
Fig. 11 shows a comparison between cyclic voltammogram (CV) of a platinum nanowire modified microelectrode array prepared in Example 27 and an unmodified microelectrode.
Fig. 12 shows a comparison between electrochemical impedance spectrum (EIS) of a platinum nanowire modified microelectrode array prepared in Example 27 and an unmodified microelectrode
Figs. 13-16 show scanning electron microscope (SEM) images of dendritic platinum modification layers of different sizes prepared in Example 40.
Fig. 17 shows a comparison between cyclic voltammogram (CV) of a dendritic platinum modified microelectrode array prepared in Example 40 and an unmodified microelectrode.
Fig. 18 shows a comparison between electrochemical impedance spectrum (EIS) of a dendritic platinum modified microelectrode array prepared in Example 40 and an unmodified microelectrode
Fig. 19 shows results of electrochemical stability test of a dendritic platinum modified microelectrode array prepared in Example 40.

### DETAILED DESCRIPTION OF THE INVENTION

Some preferred embodiments of the present invention will be described below.

In a first aspect, the present invention provides a microelectrode array having a modification layer disposed on a surface of an electrode thereof. The modification layer comprises a platinum nanopillar modification layer, a platinum nanowire modification layer or a dendritic platinum modification layer.

In a first embodiment, the platinum nanopillar modification layer, in which the platinum nanopillar may have a diameter of 50 nm to 500 nm, may have a thickness of 500 nm to 5 µm. Optionally, the platinum nanopillar modification layer has a three-dimensional nanoporous structure, which can greatly increase the surface area of the microelectrode. The platinum nanopillar modification layer is disposed by means of electrodeposition, by which the modification layer on the electrode surface can be adhered well to the electrode and is less likely to fall off.

In the first embodiment, a platinum nanopillar is used as a surface modification layer, and the modification layer can be adhered well to a substrate of the microelectrode and is less likely to fall off and cause electrode failure. The modified microelectrode has an increased surface area, reduced electrochemical impedance and increased charge injection capacity and charge storage capability of the electrode, this facilitating reducing the power consumption of an implantable system and improving the effect of electric stimulation. Further, the modification layer exhibits good biocompatibility, which greatly broadens its application in the biomedical field.

In the first embodiment, an iridium oxide layer is disposed on the surface of the platinum nanopillar modification layer, and combination of platinum nanopillar modification layer and the iridium oxide layer forms an iridium oxide/platinum nanopillar composite coating. The iridium oxide layer has a thickness of 10 nm to 500nm. Optionally, the iridium oxide layer has a thickness of 50 nm to 400 nm, or 100 nm to 300 nm. A suitable thickness of the iridium oxide layer leads to a higher charge storage capacity of the microelectrode array. Optionally, the iridium oxide/platinum nanopillar composite coating has a thickness of 500 nm to 5 µm, and further optionally 1 µm to 4 µm, or 2 µm to 3 µm. Various thicknesses result in various microelectrodes having different sized surface areas. Optionally, in the present invention, the platinum nanopillar has a diameter of 50 nm to 500 nm, and optionally, the platinum nanopillar has a diameter of 200 nm to 300 nm. A suitable diameter not only secures a good nanoporous structure of the platinum nanopillar, but also provides a suitable rough surface. Optionally, the iridium oxide/platinum nanopillar composite coating has a three-dimensional nanoporous structure, and the porous iridium oxide/platinum nanopillar composite coating has a pore diameter of 50 nm to 500 nm, further optionally 200 nm to 500 nm, or 100 nm to 300 nm. The three-dimensional nanoporous structure can greatly increase the surface area and the charge storage capacity of the microelectrode. Optionally, in the present invention, the iridium oxide/platinum nanopillar composite coating is obtained by sequentially depositing a platinum nanopillar layer and an iridium oxide layer on the surface of the electrode. The iridium oxide/platinum nanopillar composite coating, which is disposed on the surface of the electrode surface by means of electrochemical deposition, can be adhered well to the electrode and is less likely to fall off.

In the present invention, the iridium oxide/platinum nanopillar composite coating comprises a platinum nanopillar layer and an iridium oxide layer which are sequentially disposed on the surface of the electrode. As the platinum nanopillar layer has a nanoporous structure that provides a large rough surface, the platinum nanopillar layer acts as an adhesive layer between the electrode and the iridium oxide layer. Thus more iridium oxide can adhere more strongly to the electrode. Moreover, since the iridium oxide layer has ultra-high charge storage capacity, the iridium oxide/platinum nanopillar composite coating modified microelectrode array according to the invention has high charge storage capacity and high stability.

A microelectrode array is provided in the first aspect of the present invention. The microelectrode has a modification layer disposed on the surface of electrodes thereof. The modification layer can be adhered well to a substrate of the microelectrode and is less likely to fall off and cause electrode failure. The modified microelectrode has an increased surface area, reduced electrochemical impedance and increased charge injection capacity and charge storage capability of the electrode, this facilitating reducing the power consumption of an implantable system and improving the effect of electric stimulation. Further, the modification layer exhibits good biocompatibility, which greatly broadens its application in the biomedical field.

In a second aspect, the present invention provides a method for preparing a microelectrode array, wherein a modification layer is prepared on a surface of a microelectrode to be modified by means of electrodeposition to form a microelectrode array, and wherein the modification layer comprises a platinum nanopillar modification layer.

In a first embodiment, the method for preparing the microelectrode array comprises:
(1) adding an appropriate amount of a weak reducing agent to a platinum salt solution while mixing to obtain an electrodeposition solution A;
(2) forming a three-electrode system using a platinum plate as a counter electrode, Ag/AgCl as a reference electrode, a microelectrode to be modified as a working electrode, and the electrodeposition solution A, the system connecting to an electrochemical workstation; and
(3) performing electrodeposition at normal temperature and pressure for 300-600s such that a platinum nanopillar modification layer forms on the surface of the microelectrode to obtain a microelectrode array.

Optionally, the platinum salt solution is one or more selected from the group consisting of platinum nitrate, platinum chloride, chloroplatinic acid, ammonium hexachloroplatinate, sodium chloroplatinate, potassium hexachloroplatinate and potassium tetrachloroplatinate.

In the present invention, the added weak reducing agent can react with the platinum salt to accelerate the electrochemical reduction of platinum ions and the formation of elemental platinum on the surface of the microelectrode, thereby forming a more porous structure. The weak reducing agent is one or more selected from the group consisting of formic acid, hydroxylammonium chloride, citric acid, citrate, ascorbic acid, ascorbate, benzene-1,4-diol, benzene-1,2,3-triol and benzene-1,2,4-triol.

Optionally, in the electrodeposition solution A, the concentration of the platinum salt is in a range of 1-20 mmol/L, and the concentration of the weak reducing agent is in a range of 1-20 mmol/L.

Optionally, the electrodeposition comprises constant potential deposition (at a potential of -0.1V to -0.6V), constant current deposition (at a current of - 0.1µA to -0.7µA) or pulse electrodeposition (with a peak current density of 0.1A/cm<2> to 1A/cm<2>).

Optionally, the platinum nanopillar modification layer, in which the platinum nanopillar may have a diameter of 50 nm to 500 nm, may have a thickness of 500 nm to 5 µm.

Optionally, the platinum nanopillar modification layer has a three-dimensional nanoporous structure.

Optionally, the microelectrode to be modified may be a planar microelectrode, a pin microelectrode, etc.

An iridium oxide layer is prepared on the surface of the platinum nanopillar modification layer, the method of which comprises: adding an appropriate amount of an oxidizing agent and weak acid to an iridium salt solution while mixing to obtain an electrodeposition solution B; forming a three-electrode system using a platinum plate as a counter electrode, Ag/AgCl as a reference electrode, the microelectrode array provided with a platinum nanopillar modification layer as a working electrode, and the electrodeposition solution B, the system connecting to an electrochemical workstation;performing electrodeposition at normal temperature and pressure for 300-600s such that an iridium oxide layer forms on the surface of the platinum nanopillar modification layer and a combination of the platinum nanopillar modification layer and the iridium oxide layer forms an iridium oxide/platinum nanopillar composite coating.

Optionally, the iridium salt solution is one or more selected from the group consisting of chloroiridic acid, iridium oxide, iridium chloride, iridium acetylacetonate, sodium hexachloroiridate, potassium hexachloroiridate, ammonium hexachloroiridate, potassium hexanitroiridate and tetrairidium dodecacarbonyl.

Optionally, the oxidizing agent is one or more selected from the group consisting of hydrogen peroxide, oxygen, ozone, potassium peroxide and sodium peroxide.

Optionally, the weak acid is one or more selected from the group consisting of formic acid, acetic acid, oxalic acid and carbonic acid.

Optionally, in the electrodeposition solution A, the concentration of the platinum salt is in a range of 1 mmol/L to 20 mmol/L (such as 2 mmol/L, 5 mmol/L, 10 mmol/L, 15 mmol/L, 18 mmol/L), the concentration of the weak reducing agent is in a range of 1 mmol/L to 20 mmol/L (such as 2 mmol/L, 5 mmol/L, 10 mmol/L, 15 mmol/L, 18 mmol/L).

Optionally, in the electrodeposition solution B, the concentration of the iridium salt is in a range of 1 mmol/L to 5 mmol/L (such as 1 mmol/L, 2 mmol/L, 3 mmol/L, 4 mmol/L, 5 mmol/L), the concentration of the oxidizing agent is in a range of 1 mmol/L to 5 mmol/L (such as 1 mmol/L, 2 mmol/L, 3 mmol/L, 4 mmol/L, 5 mmol/L), and the concentration of the weak acid is in a range of 1 mmol/L to 5 mmol/L (such as 1 mmol/L, 2 mmol/L, 3 mmol/L, 4 mmol/L, 5 mmol/L).

Optionally, the electrodeposition comprises constant potential deposition (at a potential of -0.1V to -0.6V), constant current deposition (at a current of - 0.1µA to -0.7µA) or pulse electrodeposition (with a peak current density of 0.1A/cm<2> to 1A/cm<2>).

Optionally, the iridium oxide/platinum nanopillar composite coating has a thickness of 500 nm to 5 µm, further optionally 1 µm to 4 µm, or 2 µm to 3 µm, and the platinum nanopillar has a diameter of 50 nm to 500 nm. The iridium oxide layer has a thickness of 10 nm to 500 nm, optionally 50 nm to 400 nm, or 100 nm to 300 nm.

Optionally, the platinum nanopillar layer has a three-dimensional nanoporous structure.

Optionally, the microelectrode to be modified may be a planar microelectrode, a pin microelectrode, etc.

The second aspect of the present invention provides a method for preparing a microelectrode array, wherein the solution is simple to prepare and has no toxic substances (such as lead, etc.). The method can be easily conducted under mild condition, whereby a modification layer can be rapidly formed on the surface of the microelectrode array. The modification layer adheres well to the microelectrode substrate, which may greatly increase the surface area of the microelectrode, and enable it to have excellent electrochemical performance, thereby broadening the application of the microelectrode array in the field of nerve stimulation.

### Example 1

A method for preparing the microelectrode array comprises the following steps.
(1) At room temperature, 1L of water, 1mmol of platinum chloride (PtCl4) and 5mmol of formic acid (HCOOH) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. An electrodeposition was performed at a constant potential of -0.1V (vs Ag/AgCI) for 300-600s such that a platinum nanopillar modification layer formed on the surface of the microelectrode to obtain a platinum nanopillar modified microelectrode array.

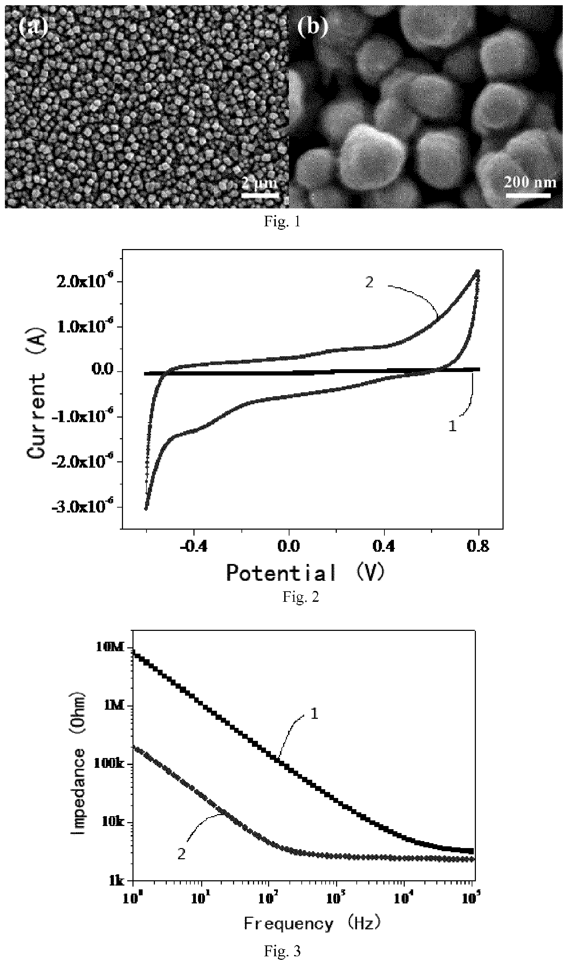

Fig. 1 shows a scanning electron microscope (SEM) image of a platinum nanopillar modification layer prepared in this embodiment. It can be seen from the figure that the obtained platinum nanopillar has a uniform morphology and a diameter of about 200 nm. Fig. 2 shows a comparison between cyclic voltammogram (CV) of the platinum nanopillar modified microelectrode array prepared in this embodiment and an unmodified microelectrode, wherein curve 1 represents an unmodified microelectrode, and curve 2 represents the platinum nanopillar modified microelectrode array of this embodiment. It can be seen from the Fig. 2 that the platinum nanopillar modification layer has a larger CV area than the unmodified microelectrode, indicating that it has superior charge storage capability. Fig. 3 shows a comparison between electrochemical impedance spectrum (EIS) of the platinum nanopillar modified microelectrode array prepared in this embodiment and an unmodified microelectrode, wherein curve 1 represents an unmodified microelectrode, and curve 2 represents the platinum nanopillar modified microelectrode array of this embodiment. It can be seen from the Fig. 3 that, compared with the unmodified microelectrode, the platinum nanopillar modification layer leads to lower electrochemical impedance, which can be as low as 2 kΩ at 1 KHz.

### Example 2

A method for preparing the microelectrode array comprises the following steps.
(1) At room temperature, 1L of water, 1mmol of platinum nitrate (Pt(NO3)4) and 10mmol of formic acid (HCOOH) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. An electrodeposition was performed at a constant potential of -0.1V (vs Ag/AgCI) for 300-600s such that a platinum nanopillar modification layer formed on the surface of the microelectrode to obtain a platinum nanopillar modified microelectrode array.

### Example 3

A method for preparing the microelectrode array comprises the following steps.
(1) At room temperature, 1L of water, 1mmol of chloroplatinic acid (H2PtCI6) and 20mmol of hydroxylammonium chloride (NH2OH·HCl) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. An electrodeposition was performed at a constant current of -0.3µA (vs Ag/AgCI) for 300-600s such that a platinum nanopillar modification layer formed on the surface of the microelectrode to obtain a platinum nanopillar modified microelectrode array.

### Example 4

A method for preparing the microelectrode array comprises the following steps.
(1) At room temperature, 1L of water, 5mmol of chloroplatinic acid (H2PtCI6) and 1mmol of formic acid (HCOOH) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. A pulse electrodeposition was performed for 300-600s with a peak current density of 2.5A/cm<2> and a pulse current duty cycle of 5 µs:500µs such that a platinum nanopillar modification layer formed on the surface of the microelectrode to obtain a platinum nanopillar modified microelectrode array.

### Example 5

A method for preparing the microelectrode array comprises the following steps.
(1) At room temperature, 1L of water, 1mmol of ammonium hexachloroplatinate ((NH4)2PtCI6) and 5mmol of sodium citrate (Na3C6H5O7) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. A pulse electrodeposition was performed for 300-600s with a peak current density of 2.5A/cm<2> and a Pulse current duty cycle of 5 µs:500µs such that a platinum nanopillar modification layer formed on the surface of the microelectrode to obtain a platinum nanopillar modified microelectrode array.

### Example 6

A method for preparing the microelectrode array comprises the following steps.
(1) At room temperature, 1L of water, 5mmol of sodium chloroplatinate (Na2PtCI6) and 10mmol of citric acid (C6H8O7) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. An electrodeposition was performed at a constant potential of -0.1V (vs Ag/AgCI) such that a platinum nanopillar modification layer formed on the surface of the microelectrode to obtain a platinum nanopillar modified microelectrode array.

### Example 7

A method for preparing the microelectrode array comprises the following steps.
(1) At room temperature, 1L of water, 5mmol of potassium hexachloroplatinate (K2PtCl6) and 20mmol of ascorbic acid (C6H8O6) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. An electrodeposition was performed at a constant potential of -0.1V (vs Ag/AgCI) such that a platinum nanopillar modification layer formed on the surface of the microelectrode to obtain a platinum nanopillar modified microelectrode array.

### Example 8

A method for preparing the microelectrode array comprises the following steps.
(1) At room temperature, 1L of water, 10mmol of potassium hexachloroplatinate (K2PtCl6) and 5mmol of formic acid (HCOOH) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. An electrodeposition was performed at a constant current of -0.3µA (vs Ag/AgCl) such that a platinum nanopillar modification layer formed on the surface of the microelectrode to obtain a platinum nanopillar modified microelectrode array.

### Example 9

A method for preparing the microelectrode array comprises the following steps.
(1) At room temperature, 1L of water, 10mmol of potassium tetrachloroplatinate (K2PtCI4) and 20mmol of formic acid (HCOOH) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. An electrodeposition was performed at a constant potential of -0.1V (vs Ag/AgCl) such that a platinum nanopillar modification layer formed on the surface of the microelectrode to obtain a platinum nanopillar modified microelectrode array.

### Example 10

A method for preparing the microelectrode array comprises the following steps.
(1) At room temperature, 1 L of water, 20mmol of potassium tetrachloroplatinate (K2PtCl4) and 5mmol of ascorbic acid (C6H8O6) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. A pulse electrodeposition was performed with a peak current density of 2.5A/cm<2> and a pulse current duty cycle of 5 µs:500µs such that a platinum nanopillar modification layer formed on the surface of the microelectrode to obtain a platinum nanopillar modified microelectrode array.

### Example 11

A method for preparing the microelectrode array comprises the following steps.
(1) At room temperature, 1L of water, 20mmol of potassium tetrachloroplatinate (K2PtCI4) and 10mmol of citric acid (C6H8O7) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. A pulse electrodeposition was performed with a peak current density of 2.5A/cm<2> and a pulse current duty cycle of 5 µs:500µs such that a platinum nanopillar modification layer formed on the surface of the microelectrode to obtain a platinum nanopillar modified microelectrode array.

### Example 12

A method for preparing the microelectrode array comprises the following steps.
(1) At room temperature, 1L of water, 20mmol of chloroplatinic acid (H2PtCI6) and 20mmol of formic acid (HCOOH) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. An electrodeposition was performed at a constant potential of -0.1V (vs Ag/AgCl) for 300-600s such that a platinum nanopillar modification layer formed on the surface of the microelectrode to obtain a platinum nanopillar modified microelectrode array.

### Example 13

A method for preparing the microelectrode array comprises the following steps.
(1) At room temperature, 1L of water, 20mmol of chloroplatinic acid (H2PtCI6) and 1mmol of formic acid (HCOOH) were added to a vessel while gently shaking and mixing to obtain an electrodeposition solution.
(2) A platinum plate, Ag/AgCl reference electrode and a microelectrode were placed in the electrodeposition solution, before connecting to an electrochemical workstation. A pulse electrodeposition was performed for 300-600s with a peak current density of 2.5A/cm<2> and a pulse current duty cycle of 5 µs:500µs such that a platinum nanopillar modification layer formed on the surface of the microelectrode to obtain a platinum nanopillar modified microelectrode array.

The platinum nanopillar modified microelectrode arrays prepared in the above Examples 2 to 13 were tested by the same method as in Example 1. The test results showed that the prepared modification layer adhered well to the microelectrode substrate, and exhibited low electrochemical impedance, large charge injection and storage capacity, good biocompatibility, and had good development potential in the field of nerve stimulation.

### Example 14

A method for preparing the microelectrode array comprises the following steps.
(1) At room temperature, 1L of water, 1mmol of platinum chloride (PtCl4) and 5mmol of formic acid (HCOOH) were added to a vessel while gently shaking and mixing to obtain a platinum nanopillar electrodeposition solution A.
(2) At room temperature, 1L of water, 1mmol of iridium chloride (lrCl4), 2mmol of hydrogen peroxide (H2O2) and 4mmol of oxalic acid (H2C2O4) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. An electrodeposition was performed at a constant potential of -0.1V (vs Ag/AgCl) for 300-600s such that a platinum nanopillar layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

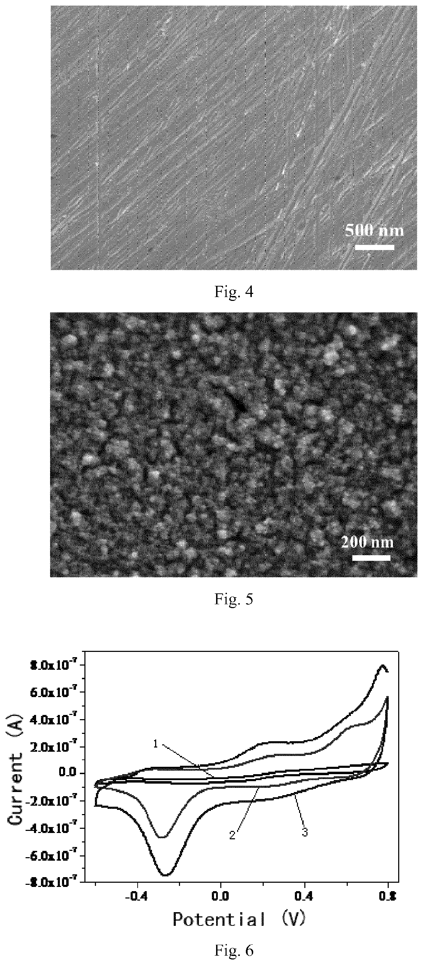

Fig. 4 shows a scanning electron microscope (SEM) image of an iridium oxide layer prepared in Example 14 of the present invention. Fig. 5 shows a scanning electron microscope (SEM) image of an iridium oxide/platinum nanopillar composite coating prepared in Example 14. It can be seen from the figures that the platinum nanopillar layer has a nanoporous surface structure, and the iridium oxide has a smooth surface. When the iridium oxide is electroplated on the surface of the platinum nanopillar to form an iridium oxide/platinum nanopillar composite coating, the composite coating is also endowed with a nanoporous surface having a porous pore diameter of 50 nm to 500 nm. In this embodiment, the iridium oxide/platinum nanopillar composite coating layer has a thickness of 500 nm to 5 µm, and the iridium oxide layer has a thickness of 10 nm to 500 nm.

Fig. 6 shows a comparison between cyclic voltammogram (CV) of an iridium oxide/platinum nanopillar composite coating modified microelectrode array prepared by Example 14, an unmodified microelectrode, and a platinum nanopillar modified microelectrode array, wherein the curve 1 represents an unmodified microelectrode, curve 2 represents the platinum nanopillar modified microelectrode array of this embodiment, and curve 3 represents the microelectrode array modified by the iridium oxide/platinum nanopillar composite coating. As can be seen from Fig. 6 , the iridium oxide/platinum nanopillar composite coating has a larger CV area than the unmodified microelectrode and the platinum nanopillar modified microelectrode array, indicating that it has superior charge storage capability.

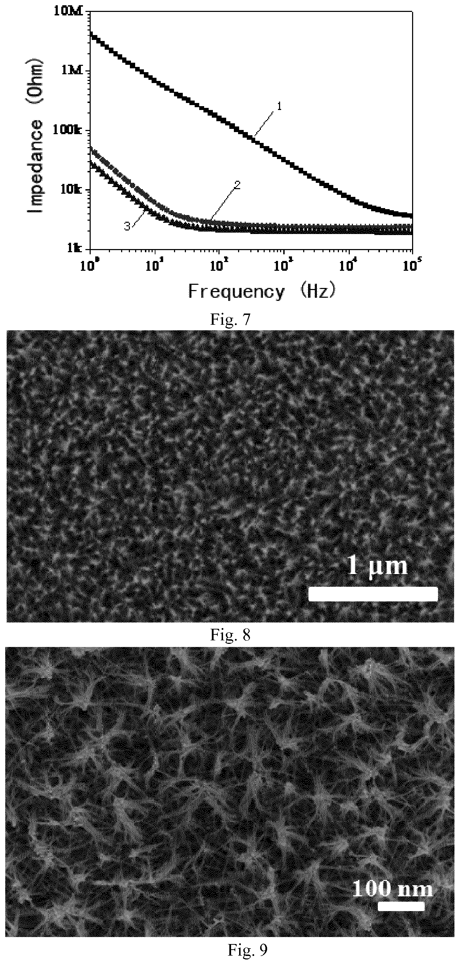

Fig. 7 shows a comparison between electrochemical impedance spectrum (EIS) of an iridium oxide/platinum nanopillar composite coating modified microelectrode array prepared by Example 14, an unmodified microelectrode, and a platinum nanopillar modified microelectrode array, wherein curve 1 represents the unmodified microelectrode, curve 2 represents the platinum nanopillar modified microelectrode array of this embodiment, and curve 3 represents the iridium oxide/platinum nanopillar composite coating of this embodiment. As can be seen from Fig. 7 , compared with the unmodified microelectrode and the platinum nanopillar modified microelectrode array, the iridium oxide/platinum nanopillar composite coating of this embodiment leads to lower electrochemical impedance, which can be as low as 2 kΩ at 1 KHz.

### Example 15

A method for preparing the microelectrode array comprises the following steps.
(1) At room temperature, 1L of water, 5mmol of ammonium hexachloroplatinate ((NH4)2PtCI6) and 5mmol of sodium citrate (Na3C6H5O7) were added to a vessel while gently shaking and mixing to obtain a platinum nanopillar electrodeposition solution A.
(2) At room temperature, 1L of water, 3mmol of iridium chloride (lrCl4), 5mmol of sodium hexachloroiridate (Cl6H12lrNa2O6) and 4mmol of formic acid (HCOOH) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. A pulse electrodeposition was performed for 300-600s with a peak current density of 2.5A/cm<2> and a pulse current duty cycle of 5 µs:500µs for 300-600s such that a platinum nanopillar modification layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

In this embodiment, the iridium oxide/platinum nanopillar composite coating had a thickness of 1 µm to 5 µm, the platinum nanopillar had a diameter of 200 to 300 nm, the iridium oxide layer had a thickness of 10 nm to 500 nm. The porous iridium oxide/platinum nanopillar composite coating had a pore diameter of 50 nm to 500 nm

### Example 16

A method for preparing the microelectrode array comprises the following steps.
(1) At room temperature, 1L of water, 1mmol of platinum nitrate (Pt(NO3)4) and 10mmol of formic acid (HCOOH) were added to a vessel while gently shaking and mixing to obtain a platinum nanopillar electrodeposition solution A.
(2) At room temperature, 1L of water, 3mmol of ammonium hexachloroiridate (Cl6H15lrN2O6), 5mmol of potassium peroxide (K2O2) and 4mmol of acetic acid (CH3COOH) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. An electrodeposition was performed at a constant current of -0.3µA (vs Ag/AgCl) for 300-600s such that a platinum nanopillar modification layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

In this embodiment, the iridium oxide/platinum nanopillar composite coating had a thickness of 2-4 µm, the platinum nanopillar had a diameter of 200-400 nm, the iridium oxide layer had a thickness of 200-500 nm. The porous iridium oxide/platinum nanopillar composite coating had a pore diameter of 200-500 nm.

### Example 17

A method for preparing the microelectrode array comprises the following steps.
(1) At room temperature, 1L of water, 5mmol of potassium hexachloroplatinate (K2PtCl6) and 20mmol of ascorbic acid (C6H8O6) were added to a vessel while gently shaking and mixing to obtain a platinum nanopillar electrodeposition solution A
(2) At room temperature, 1L of water, 3mmol of iridium chloride (lrCl4), 5mmol of sodium peroxide (Na2O2) and 4mmol of oxalic acid (H2C2O4) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. An electrodeposition was performed at a constant potential of -0.1V (vs Ag/AgCl) for 300-600s such that a platinum nanopillar modification layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

In this embodiment, the iridium oxide/platinum nanopillar composite coating had a thickness of 500nm to 2 µm, the platinum nanopillar had a diameter of 200-300 nm, the iridium oxide layer had a thickness of 200-400 nm. The porous iridium oxide/platinum nanopillar composite coating had a pore diameter of 100-300 nm.

### Example 18

A method for preparing the microelectrode array comprises the following steps.
(1) At room temperature, 1L of water, 10mmol of potassium tetrachloroplatinate (K2PtCl4) and 20mmol of formic acid (HCOOH) were added to a vessel while gently shaking and mixing to obtain a platinum nanopillar electrodeposition solution A.
(2) At room temperature, 1 L of water, 3mmol of iridium acetylacetonate lr(O2C5H7)3, 5mmol of hydrogen peroxide (H2O2) and 4mmol of oxalic acid (H2C2O4) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. A pulse electrodeposition was performed with a peak current density of 2.5A/cm<2> and a pulse current duty cycle of 5 µs:500µs such that a platinum nanopillar modification layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

In this embodiment, the iridium oxide/platinum nanopillar composite coating had a thickness of 1-3 µm, the platinum nanopillar had a diameter of 50-200 nm, the iridium oxide layer had a thickness of 10-200 nm. The porous iridium oxide/platinum nanopillar composite coating had a pore diameter of 50-200 nm.

### Example 19

A method for preparing the microelectrode array comprises the following steps.
(1) At room temperature, 1L of water, 20mmol of chloroplatinic acid (H2PtCI6) and 20mmol of acetic acid (CH3COOH) were added to a vessel while gently shaking and mixing to obtain a platinum nanopillar electrodeposition solution A.
(2) At room temperature, 1 L of water, 3mmol of chloroiridic acid (H2IrCl6·6H2O), 5mmol of sodium peroxide (Na2O2) and 4mmol of formic acid (HCOOH) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. An electrodeposition was performed at a constant potential of -0.1V (vs Ag/AgCl) for 300-600s such that a platinum nanopillar modification layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

In this embodiment, the iridium oxide/platinum nanopillar composite coating had a thickness of 500nm to 5µm, the platinum nanopillar had a diameter of 200-300nm, the iridium oxide layer had a thickness of 10-500 nm. The porous iridium oxide/platinum nanopillar composite coating had a pore diameter of 50-500 nm

### Example 20

A method for preparing the microelectrode array comprises the following steps.
(1) At room temperature, 1L of water, 1mmol of platinum nitrate (Pt(NO3)4) and 10mmol of formic acid (HCOOH) were added to a vessel while gently shaking and mixing to obtain a platinum nanopillar electrodeposition solution A.
(2) At room temperature, 1L of water, 3mmol of potassium hexanitroiridate (IrK3N6O12), 5mmol of sodium peroxide (Na2O2) and 4mmol of acetic acid (CH3COOH) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. An electrodeposition was performed at a constant current of -0.3µA (vs Ag/AgCl) for 300-600s such that a platinum nanopillar modification layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

In this embodiment, the iridium oxide/platinum nanopillar composite coating had a thickness of 500nm to 5µm, the platinum nanopillar had a diameter of 50-500nm, the iridium oxide layer had a thickness of 10-500 nm. The porous iridium oxide/platinum nanopillar composite coating had a pore diameter of 50-500 nm.

### Example 21

A method for preparing the microelectrode array comprises the following steps.
(1) At room temperature, 1L of water, 5mmol of ammonium hexachloroplatinate ((NH4)2PtCI6) and 5mmol of sodium citrate (Na3C6H5O7) were added to a vessel while gently shaking and mixing to obtain a platinum nanopillar electrodeposition solution A.
(2) At room temperature, 1L of water, 3mmol of tetrairidium dodecacarbonyl (Ir4(CO)12), 5mmol of hydrogen peroxide (H2O2) and 4mmol of citric acid (C6H8O7) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. An electrodeposition was performed at a constant current of -0.3µA (vs Ag/AgCl) for 300-600s such that a platinum nanopillar modification layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

In this embodiment, the iridium oxide/platinum nanopillar composite coating had a thickness of 500nm to 5µm, the platinum nanopillar had a diameter of 50-500nm, the iridium oxide layer had a thickness of 10-500 nm. The porous iridium oxide/platinum nanopillar composite coating had a pore diameter of 50-500 nm.

### Example 22

A method for preparing the microelectrode array comprises the following steps.
(1) At room temperature, 1L of water, 5mmol of sodium chloroplatinate (Na2PtCI6) and 10mmol of citric acid (C6H8O7) were added to a vessel while gently shaking and mixing to obtain a platinum nanopillar electrodeposition solution A.
(2) At room temperature, 1L of water, 3mmol of sodium hexachloroiridate (Cl6H12IrNa2O6), 5mmol of potassium peroxide (K2O2) and 4mmol of oxalic acid (H2C2O4) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. An electrodeposition was performed at a constant potential of -0.1V (vs Ag/AgCl) such that a platinum nanopillar modification layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

In this embodiment, the iridium oxide/platinum nanopillar composite coating had a thickness of 500nm to 5µm, the platinum nanopillar had a diameter of 50-500nm, the iridium oxide layer had a thickness of 10-500 nm. The porous iridium oxide/platinum nanopillar composite coating had a pore diameter of 50-500 nm

### Example 23

A method for preparing the microelectrode array comprises the following steps.
(1) At room temperature, 1 L of water, 5mmol of potassium hexachloroplatinate (K2PtCl6) and 20mmol of ascorbic acid (C6H8O6) were added to a vessel while gently shaking and mixing to obtain a platinum nanopillar electrodeposition solution A.
(2) At room temperature, 1L of water, 3mmol of iridium chloride (lrCl4), 5mmol of hydrogen peroxide (H2O2) and 4mmol of citric acid (C6H8O7) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. An electrodeposition was performed at a constant potential of -0.1V (vs Ag/AgCl) such that a platinum nanopillar modification layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

In this embodiment, the iridium oxide/platinum nanopillar composite coating had a thickness of 500nm to 5µm, the platinum nanopillar had a diameter of 50-500nm, the iridium oxide layer had a thickness of 10-500 nm. The porous iridium oxide/platinum nanopillar composite coating had a pore diameter of 50-500 nm.

### Example 24

A method for preparing the microelectrode array comprises the following steps.
(1) At room temperature, 1L of water, 10mmol of potassium tetrachloroplatinate (K2PtCl4) and 20mmol of formic acid (HCOOH) were added to a vessel while gently shaking and mixing to obtain a platinum nanopillar electrodeposition solution A
(2) At room temperature, 1L of water, 3mmol of sodium hexachloroiridate (Cl6H12IrNa2O6), 5mmol of hydrogen peroxide (H2O2) and 4mmol of oxalic acid (H2C2O4) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. An electrodeposition was performed at a constant potential of -0.1V (vs Ag/AgCl) such that a platinum nanopillar modification layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

In this embodiment, the iridium oxide/platinum nanopillar composite coating had a thickness of 500nm to 5µm, the platinum nanopillar had a diameter of 50-500nm, the iridium oxide layer had a thickness of 10-500 nm. The porous iridium oxide/platinum nanopillar composite coating had a pore diameter of 50-500 nm.

### Example 25

A method for preparing the microelectrode array comprises the following steps.
(1) At room temperature, 1 L of water, 20mmol of potassium tetrachloroplatinate (K2PtCl4) and 5mmol of ascorbic acid (C6H8O6) were added to a vessel while gently shaking and mixing to obtain a platinum nanopillar electrodeposition solution A
(2) At room temperature, 1L of water, 3mmol of sodium hexachloroiridate (Cl6H12IrNa2O6), 5mmol of potassium peroxide (K2O2) and 4mmol of citric acid (C6H8O7) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. A pulse electrodeposition was performed with a peak current density of 2.5A/cm<2> and a pulse current duty cycle of 5 µs:500µs such that a platinum nanopillar modification layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

In this embodiment, the iridium oxide/platinum nanopillar composite coating had a thickness of 500nm to 5µm, the platinum nanopillar had a diameter of 50-500nm, the iridium oxide layer had a thickness of 10-500 nm. The porous iridium oxide/platinum nanopillar composite coating had a pore diameter of 50-500 nm.

### Example 26

A method for preparing the microelectrode array comprises the following steps.
(1) At room temperature, 1L of water, 1mmol of chloroplatinic acid (H2PtCI6) and 20mmol of hydroxylammonium chloride (NH2OH·HCl) were added to a vessel while gently shaking and mixing to obtain a platinum nanopillar electrodeposition solution A.
(2) At room temperature, 1L of water, 3mmol of iridium chloride (lrCl4), 5mmol of potassium peroxide (K2O2) and 4mmol of acetic acid (CH3COOH) were added to a vessel while gently shaking and mixing to obtain an iridium oxide electrodeposition solution B.
(3) A platinum plate, Ag/AgCl reference electrode and a microelectrode to be modified were placed in the platinum nanopillar electrodeposition solution A, before connecting to an electrochemical workstation. A pulse electrodeposition was performed with a peak current density of 2.5A/cm<2> and a pulse current duty cycle of 5 µs:500µs such that a platinum nanopillar modification layer formed on the surface of the microelectrode to obtain a platinum nanopillar modification layer modified microelectrode array.
(4) A platinum plate, Ag/AgCl reference electrode and the platinum nanopillar modification layer modified microelectrode were placed in the iridium oxide electrodeposition solution B, before connecting to an electrochemical workstation. An electrodeposition was performed at a constant potential of 0.5V (vs Ag/AgCl) for 300-600s such that an iridium oxide/platinum nanopillar composite coating formed on the surface of the microelectrode to obtain an iridium oxide/platinum nanopillar composite coating modified microelectrode array.

In this embodiment, the iridium oxide/platinum nanopillar composite coating had a thickness of 500nm to 5µm, the platinum nanopillar had a diameter of 50-500nm, the iridium oxide layer had a thickness of 10-500 nm. The porous iridium oxide/platinum nanopillar composite coating had a pore diameter of 50-500 nm.

The platinum nanopillar modified microelectrode arrays prepared in the above Examples 15 to 26 were tested by the same method as in Example 14. The test results showed that the prepared modification layer adhered well to the microelectrode substrate, and exhibited low electrochemical impedance, large charge injection and storage capacity, good biocompatibility, and had good development potential in the field of nerve stimulation.

### Example 27

A method for preparing the microelectrode array comprises the following steps.
(1) At room temperature, 1L of water, 1mmol of platinum chloride (PtCl4) and 6mmol of formic acid (HCOOH) were added to a vessel while gently shaking and mixing to obtain a chemical deposition solution.
(2) A microelectrode to be modified was placed in the chemical deposition solution and stored at room temperature for 36 hours, followed by placing the microelectrode in water for ultrasonically treating at a power of 600W and a frequency of 40KHz for 30 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

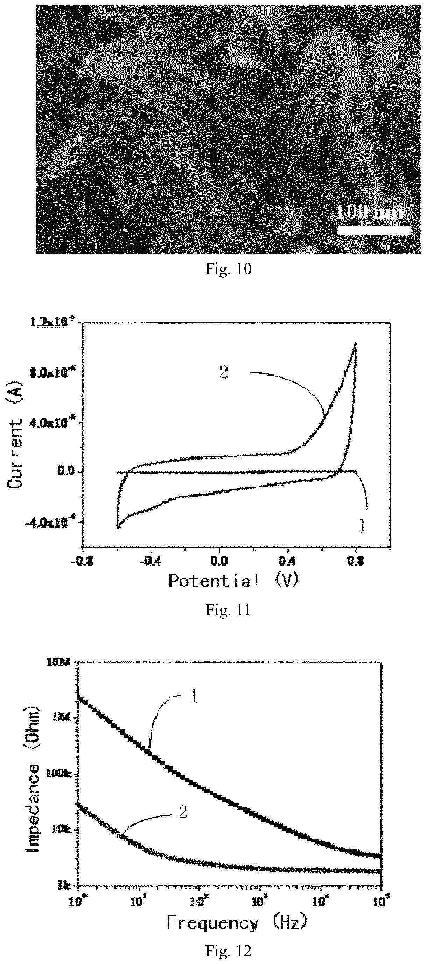

Figs. 8, 9 and 10 show a scanning electron microscope (SEM) image of a platinum nanowire modification layer prepared in Example 27, at different magnifications. It can be seen from Fig. 8 that the obtained platinum nanowire modification layer has uniform morphology without impurities on the surface. As can be seen from Fig. 9 , the obtained platinum nanowire modification layer has a highly porous and rough surface, and has a large surface area. It can be seen from the Fig. 10 that the obtained platinum nanowire has a uniform morphology and a diameter of about 8 nm.

Fig. 11 shows a comparison between cyclic voltammogram (CV) of a platinum nanowire modified microelectrode array prepared in Example 27 of the present invention and an unmodified microelectrode, wherein curve 1 represents an unmodified microelectrode, and curve 2 represents the platinum nanowire modified microelectrode array of this embodiment. It can be seen from the Fig. 11 that the platinum nanowire modification layer has a larger CV area than the unmodified microelectrode, indicating that it has superior charge storage capability. Fig. 12 shows a comparison between electrochemical impedance spectrum (EIS) of a platinum nanowire modified microelectrode array prepared in Example 27 and an unmodified microelectrode, wherein curve 1 represents an unmodified microelectrode, and curve 2 represents the platinum nanowire modified microelectrode array of this embodiment. It can be seen from the Fig. 12 that, compared with the unmodified microelectrode, the platinum nanowire modification layer leads to lower electrochemical impedance, which can be as low as 1 kΩ at 1 KHz.

### Example 28

A method for preparing the microelectrode array comprises the following steps.
(1) At room temperature, 1L of water, 2mmol of platinum nitrate (Pt(NO3)4) and 8mmol of formic acid (HCOOH) were added to a vessel while gently shaking and mixing to obtain a chemical deposition solution.
(2) A microelectrode to be modified was placed in the chemical deposition solution and stored at room temperature for 36 hours, followed by placing the microelectrode in water for ultrasonically treating at a power of 600W and a frequency of 40KHz for 50 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

### Example 29

A method for preparing the microelectrode array comprises the following steps.
(1) At room temperature, 1L of water, 5mmol of ammonium hexachloroplatinate ((NH4)2PtCI6) and 10mmol of sodium citrate (Na3C6H5O7) were added to a vessel while gently shaking and mixing to obtain a chemical deposition solution.
(2) A microelectrode to be modified was placed in the chemical deposition solution and stored at room temperature for 36 hours, followed by placing the microelectrode in water for ultrasonically treating at a power of 600W and a frequency of 40KHz for 10 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

### Example 30

A method for preparing the microelectrode array comprises the following steps.
(1) At room temperature, 1L of water, 14mmol of chloroplatinic acid (H2PtCI6) and 1mmol of formic acid (HCOOH) were added to a vessel while gently shaking and mixing to obtain a chemical deposition solution.
(2) A microelectrode to be modified was placed in the chemical deposition solution and stored at room temperature for 12 hours, followed by placing the microelectrode in water for ultrasonically treating at a power of 600W and a frequency of 40KHz for 20 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

### Example 31

A method for preparing the microelectrode array comprises the following steps.
(1) At room temperature, 1L of water, 10mmol of sodium chloroplatinate (Na2PtCI6) and 5mmol of citric acid (C6H8O7) were added to a vessel while gently shaking and mixing to obtain a chemical deposition solution.
(2) A microelectrode to be modified was placed in the chemical deposition solution and stored at room temperature for 36 hours, followed by placing the microelectrode in water for ultrasonically treating at a power of 600W and a frequency of 40KHz for 30 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

### Example 32

A method for preparing the microelectrode array comprises the following steps.
(1) At room temperature, 1L of water, 8mmol of chloroplatinic acid (H2PtCI6) and 8mmol of hydroxylammonium chloride (NH2OH·HCl) were added to a vessel while gently shaking and mixing to obtain a chemical deposition solution.
(2) A microelectrode to be modified was placed in the chemical deposition solution and stored at room temperature for 36 hours, followed by placing the microelectrode in water for ultrasonically treating at a power of 600W and a frequency of 40KHz for 30 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

### Example 33

A method for preparing the microelectrode array comprises the following steps.
(1) At room temperature, 1 L of water, 20mmol of potassium tetrachloroplatinate (K2PtCl4) and 20mmol of formic acid (HCOOH) were added to a vessel while gently shaking and mixing to obtain a chemical deposition solution.
(2) A microelectrode to be modified was placed in the chemical deposition solution and stored at room temperature for 36 hours, followed by placing the microelectrode in water for ultrasonically treating at a power of 600W and a frequency of 40KHz for 30 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

### Example 34

A method for preparing the microelectrode array comprises the following steps.
(1) At room temperature, 1L of water, 1mmol of potassium tetrachloroplatinate (K2PtCl4) and 1mmol of ascorbic acid (C6H8O6) were added to a vessel while gently shaking and mixing to obtain a chemical deposition solution.
(2) A microelectrode to be modified was placed in the chemical deposition solution and stored at room temperature for 36 hours, followed by placing the microelectrode in water for ultrasonically treating at a power of 600W and a frequency of 40KHz for 30 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

### Example 35

A method for preparing the microelectrode array comprises the following steps.
(1) At room temperature, 1L of water, 15mmol of potassium hexachloroplatinate (K2PtCl6) and 1mmol of citric acid (C6H8O7) were added to a vessel while gently shaking and mixing to obtain a chemical deposition solution.
(2) A microelectrode to be modified was placed in the chemical deposition solution and stored at room temperature for 36 hours, followed by placing the microelectrode in water for ultrasonically treating at a power of 600W and a frequency of 40KHz for 30 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

### Example 36

A method for preparing the microelectrode array comprises the following steps.
(1) At room temperature, 1L of water, 20mmol of chloroplatinic acid (H2PtCI6) and 15mmol of formic acid (HCOOH) were added to a vessel while gently shaking and mixing to obtain a chemical deposition solution.
(2) A microelectrode to be modified was placed in the chemical deposition solution and stored at room temperature for 36 hours, followed by placing the microelectrode in water for ultrasonically treating at a power of 600W and a frequency of 40KHz for 30 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

### Example 37

A method for preparing the microelectrode array comprises the following steps.
(1) At room temperature, 1L of water, 20mmol of chloroplatinic acid (H2PtCI6) and 20mmol of ascorbic acid (C6H8O6) were added to a vessel while gently shaking and mixing to obtain a chemical deposition solution.
(2) A microelectrode to be modified was placed in the chemical deposition solution and stored at room temperature for 36 hours, followed by placing the microelectrode in water for ultrasonically treating at a power of 600W and a frequency of 40KHz for 30 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

### Example 38

A method for preparing the microelectrode array comprises the following steps.
(1) At room temperature, 1L of water, 10mmol of potassium hexachloroplatinate (K2PtCI6) and 5mmol of formic acid (HCOOH) were added to a vessel while gently shaking and mixing to obtain a chemical deposition solution.
(2) A microelectrode to be modified was placed in the chemical deposition solution and stored at room temperature for 24 hours, followed by placing the microelectrode in water for ultrasonically treating at a power of 600W and a frequency of 40KHz for 30 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

### Example 39

A method for preparing the microelectrode array comprises the following steps.
(1) At room temperature, 1 L of water, 20mmol of potassium tetrachloroplatinate (K2PtCl4) and 10mmol of citric acid (C6H8O7) were added to a vessel while gently shaking and mixing to obtain a chemical deposition solution.
(2) A microelectrode to be modified was placed in the chemical deposition solution and stored at room temperature for 36 hours, followed by placing the microelectrode in water for ultrasonically treating at a power of 600W and a frequency of 40KHz for 50 minutes to remove platinum nanowires poorly adhered to the substrate of electrode, thereby on the surface of microelectrode forming a platinum nanowire modification layer which exhibits good biocompatibility, large surface area and good adherent performance, and thus obtaining a platinum nanowire modified microelectrode array.

The platinum nanowire modified microelectrode arrays prepared in the above Examples 28 to 39 were tested by the same method as in Example 27. The test results showed that the prepared platinum nanowire modification layer adhered well to the microelectrode substrate, and exhibited low electrochemical impedance, large charge injection and storage capacity, good biocompatibility, and had good development potential in the field of nerve stimulation.

### Example 40

A method for preparing the microelectrode array comprises the following steps.
(1) At room temperature, 1L of water, 1mmol of platinum chloride (PtCl4) and 5mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. An electrodeposition was performed at a constant potential of -0.3V (vs Ag/AgCl) and a temperature of 35°C for 3000s such that a dendritic platinum modification layer formed on the surface of the microelectrode to obtain a dendritic platinum modified microelectrode array.

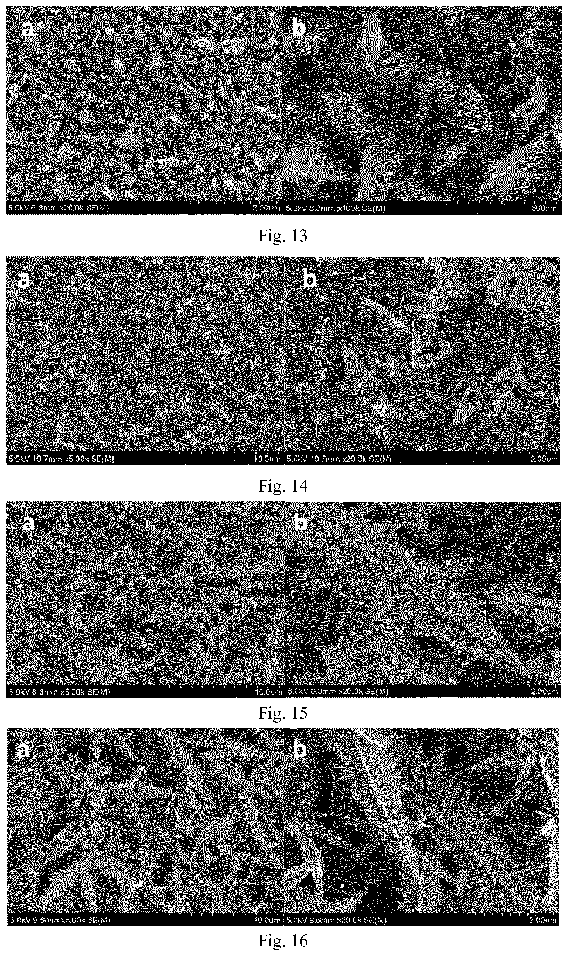

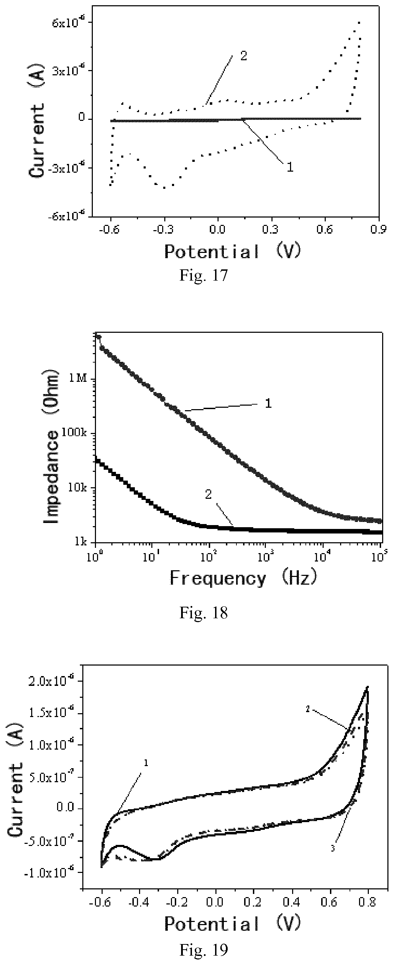

Fig. 13 shows a scanning electron microscope (SEM) image of dendritic platinum modification layers prepared in this embodiment. As can be seen from the figure, after modification, a dendritic platinum having a dendritic structure formed on the surface of the microelectrode. Figs. 14-16 show scanning electron microscope (SEM) images of dendritic platinum modification layers of different sizes. Adjusting parameters such as deposition time and deposition temperature will lead to dendritic platinum of different sizes. As shown in Figs. 14-16 , the electrodeposition was performed for 4000 s, 5000 s and 6000 s, respectively, and at a temperature of 40 °C, 50 °C and 60 °C, respectively. Fig. 17 shows a comparison between cyclic voltammogram (CV) of a dendritic platinum modified microelectrode array prepared in Example 40 and an unmodified microelectrode, wherein curve 1 represents an unmodified microelectrode, and curve 2 represents the dendritic platinum modified microelectrode array of this embodiment. It can be seen from the Fig. 17 that the dendritic platinum modification layer has an increased CV about 44 times of the unmodified microelectrode (increased from 3.7 mC/cm<2> to 162.4mC/cm<2>), indicating that it has superior charge storage capability. Fig. 18 shows a comparison between electrochemical impedance spectrum (EIS) of a dendritic platinum modified microelectrode array prepared in Example 40 and an unmodified microelectrode, wherein curve 1 represents an unmodified microelectrode, and curve 2 represents the dendritic platinum modified microelectrode array of this embodiment. It can be seen from the Fig. 18 that, compared with the unmodified microelectrode, the dendritic platinum modification layer leads to lower electrochemical impedance, which can be as low as 1.3 kΩ at 1 KHz. Fig. 19 shows results of electrochemical stability test of a dendritic platinum modified microelectrode array prepared in Example 40 of the present invention. A long-term high-frequency pulse experiments were performed on a microelectrode modified with dendritic platinum. The change of the charge storage capacity before and after the pulse experiments (the CV closure curve The change in the integral area of the cathode portion) was calculated. The curve 1 represents the dendritic platinum modification layer which was not subjected to the pulse, and the curve 2 represents the dendritic platinum modification layer which was subjected to 48-hour uninterrupted high frequency pulse, and curve 3 represents the dendritic platinum modification layer which was subjected to 96-hour uninterrupted high frequency pulse. It can be seen from Fig. 19 that after long-term high frequency pulse treatment (more than 2x10<6> times), charge storage capacity of the dendritic platinum modification layer has only a small loss (less than 2%), indicating that the dendritic platinum modification layer prepared in the embodiment has excellent long-term stability.

The microelectrodes used in this embodiment were prepared by a series of micromachining processes such as photolithography (EVG 610 UV exposure machine, Austria), sputtering, etching, etc.

### Example 41

A method for preparing the microelectrode array comprises the following steps.
(1) At room temperature, 1L of water, 1mmol of platinum nitrate (Pt(NO3)4) and 10mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. An electrodeposition was performed at a constant potential of -0.5V (vs Ag/AgCl) and a temperature of 50°C for 5000s such that a dendritic platinum modification layer formed on the surface of the microelectrode to obtain a dendritic platinum modified microelectrode array.

### Example 42

A method for preparing the microelectrode array comprises the following steps.
(1) At room temperature, 1L of water, 1mmol of chloroplatinic acid (H2PtCI6) and 8mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. An electrodeposition was performed at a constant current of -1.0µA (vs Ag/AgCl) and a temperature of 60°C for 3000s such that a dendritic platinum modification layer formed on the surface of the microelectrode to obtain a dendritic platinum modified microelectrode array.

### Example 43

A method for preparing the microelectrode array comprises the following steps.
(1) At room temperature, 1L of water, 5mmol of chloroplatinic acid (H2PtCI6) and 1mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. A pulse electrodeposition was performed with a peak current density of 2.5A/cm<2> and a pulse current duty cycle of 5 µs:500µs and a temperature of 55°C for 3000s such that a dendritic platinum modification layer formed on the surface of the microelectrode to obtain a dendritic platinum modified microelectrode array.

### Example 44

A method for preparing the microelectrode array comprises the following steps.
(1) At room temperature, 1L of water, 5mmol of ammonium hexachloroplatinate ((NH4)2PtCI6) and 5mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. A pulse electrodeposition was performed with a peak current density of 1.5A/cm<2> and a pulse current duty cycle of 5 µs:500µs and a temperature of 60°C for 5000s such that a dendritic platinum modification layer formed on the surface of the microelectrode to obtain a dendritic platinum modified microelectrode array.

### Example 45

A method for preparing the microelectrode array comprises the following steps.
(1) At room temperature, 1L of water, 5mmol of sodium chloroplatinate (Na2PtCI6) and 10mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. An electrodeposition was performed at a constant potential of -0.8V (vs Ag/AgCl) and a temperature of 45°C for 3000s such that a dendritic platinum modification layer formed on the surface of the microelectrode to obtain a dendritic platinum modified microelectrode array.

### Example 46

A method for preparing the microelectrode array comprises the following steps.
(1) At room temperature, 1L of water, 5mmol of potassium hexachloroplatinate (K2PtCl6) and 20mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. An electrodeposition was performed at a constant potential of -0.6V (vs Ag/AgCl) and a temperature of 45°C for 4000s such that a dendritic platinum modification layer formed on the surface of the microelectrode to obtain a dendritic platinum modified microelectrode array.

### Example 47

A method for preparing the microelectrode array comprises the following steps.
(1) At room temperature, 1L of water, 10mmol of potassium hexachloroplatinate (K2PtCl6) and 5mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. An electrodeposition was performed at a constant current of -0.5µA (vs Ag/AgCl) and a temperature of 50°C for 6000s such that a dendritic platinum modification layer formed on the surface of the microelectrode to obtain a dendritic platinum modified microelectrode array.

### Example 48

A method for preparing the microelectrode array comprises the following steps.
(1) At room temperature, 1L of water, 10mmol of potassium tetrachloroplatinate (K2PtCl4) and 20mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. An electrodeposition was performed at a constant current of -1.5µA (vs Ag/AgCl) and a temperature of 40°C for 5000s such that a dendritic platinum modification layer formed on the surface of the microelectrode to obtain a dendritic platinum modified microelectrode array.

### Example 49

A method for preparing the microelectrode array comprises the following steps.
(1) At room temperature, 1L of water, 20mmol of potassium tetrachloroplatinate (K2PtCl4) and 5mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. A pulse electrodeposition was performed with a peak current density of 0.8A/cm<2> and a pulse current duty cycle of 5 µs:500µs and a temperature of 60°C for 6000s such that a dendritic platinum modification layer formed on the surface of the microelectrode to obtain a dendritic platinum modified microelectrode array.

### Example 50

A method for preparing the microelectrode array comprises the following steps.
(1) At room temperature, 1 L of water, 20mmol of potassium tetrachloroplatinate (K2PtCl4) and 10mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. A pulse electrodeposition was performed with a peak current density of 2.5A/cm<2> and a pulse current duty cycle of 5 µs:500µs and a temperature of 40°C for 5000s such that a dendritic platinum modification layer formed on the surface of the microelectrode to obtain a dendritic platinum modified microelectrode array.

### Example 51

A method for preparing the microelectrode array comprises the following steps.
(1) At room temperature, 1L of water, 20mmol of chloroplatinic acid (H2PtCI6) and 20mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. An electrodeposition was performed at a constant potential of -0.3V (vs Ag/AgCl) and a temperature of 50°C for 6000s such that a dendritic platinum modification layer formed on the surface of the microelectrode to obtain a dendritic platinum modified microelectrode array.

### Example 52

A method for preparing the microelectrode array comprises the following steps.
(1) At room temperature, 1L of water, 20mmol of chloroplatinic acid (H2PtCI6) and 1mmol of formic acid (HCOOH) were added to a vessel while gently shaking and mixing, followed by supplying argon gas for 30 minutes to obtain an electrodeposition solution C.
(2) A platinum plate, Ag/AgCl reference electrode and a microelectrode were placed in the electrodeposition solution C, before connecting to an electrochemical workstation. An electrodeposition was performed at a constant potential of -0.8V (vs Ag/AgCl) and a temperature of 40°C for 3000s such that a dendritic platinum modification layer formed on the surface of the microelectrode to obtain a dendritic platinum modified microelectrode array.

The dendritic platinum modified microelectrode arrays prepared in the above Examples 41 to 52 were tested by the same method as in Example 40. The test results showed that the prepared modification layer adhered well to the microelectrode substrate, and exhibited low electrochemical impedance, large charge injection and storage capacity, good biocompatibility, and had good development potential in the field of nerve stimulation.

## Claims

1. A microelectrode array having a modification layer disposed on a surface of a microelectrode thereof, wherein the modification layer comprises a platinum nanopillar modification layer disposed by means of electrodeposition, **characterised in that** an iridium oxide layer disposed by means of electrodeposition is provided 2. on a surface of the platinum nanopillar modification layer; and wherein the platinum nanopillar modification layer and the iridium oxide layer form an iridium oxide/platinum nanopillar composite coating, and wherein the iridium oxide layer has a thickness of 10 nm to 500 nm.

2. . The microelectrode array according to claim 1, wherein the platinum nanopillar modification layer has a thickness of 500 nm to 5 µm, and the platinum nanopillar of the platinum nanopillar modification layer has a diameter of 50 nm to 500 nm.

3. . The microelectrode array according to claim 1, wherein the iridium oxide/platinum nanopillar composite coating has a three-dimensional nanoporous structure, and the porous iridium oxide/platinum nanopillar composite coating has a pore diameter of 50 nm to 500 nm.

4. . A method for preparing a microelectrode array, wherein a modification layer is prepared on a surface of a microelectrode to be modified by means of electrodeposition to form the microelectrode array, wherein the modification layer comprises a platinum nanopillar modification layer, **characterised in that** the method comprises:
(1) adding an appropriate amount of a weak reducing agent to a platinum salt solution while mixing to obtain an electrodeposition solution A;
(2) forming a first three-electrode system using a platinum plate as a counter electrode, Ag/AgCl as a reference electrode, the microelectrode to be modified as a working electrode, and the electrodeposition solution A, the first three-electrode system connecting to an electrochemical workstation;
(3) performing electrodeposition at normal temperature and pressure for 300-600s such that a platinum nanopillar modification layer forms on the surface of the microelectrode to obtain the microelectrode array, wherein an iridium oxide layer is prepared on a surface of the platinum nanopillar modification layer, the method further comprising: adding an appropriate amount of an oxidizing agent and weak acid to an iridium salt solution while mixing to obtain an electrodeposition solution B; forming a second three-electrode system using a platinum plate as a counter electrode Ag/AgCl as a reference electrode, the microelectrode array provided with the platinum nanopillar modification layer as a working electrode, and the electrodeposition solution B, the second three-electrode system connecting to an electrochemical workstation; and performing electrodeposition at normal temperature and pressure for 300-600s such that the iridium oxide layer having a thickness of 10 nm to 500 nm forms on the surface of the platinum nanopillar modification layer and a combination of the platinum nanopillar modification layer and the iridium oxide layer forms an iridium oxide/platinum nanopillar composite coating.

5. . The method for preparing a microelectrode array according to claim 4, wherein in the electrodeposition solution B, the concentration of the iridium salt is in a range of 1 mmol/L to 5 mmol/L, the concentration of the oxidizing agent is in a range of 1 mmol/L to 5 mmol/L, and the concentration of the weak acid is in a range of 1 mmol/L to 5 mmol/L.

6. . The method for preparing a microelectrode array according to claim 4 ,wherein the platinum salt solution is one or more selected from the group consisting of platinum nitrate, platinum chloride, chloroplatinic acid, ammonium hexachloroplatinate, sodium chloroplatinate, potassium hexachloroplatinate and potassium tetrachloroplatinate.

7. . The method for preparing a microelectrode array according to claim 4, wherein the weak reducing agent is one or more selected from the group consisting of formic acid, hydroxylammonium chloride, citric acid, citrate, ascorbic acid, ascorbate, benzene-1,4-diol, benzene-1,2,3-triol and benzene-1,2,4-triol.

8. . The method for preparing a microelectrode array according to claim 4, wherein in the electrodeposition solution A, the concentration of the platinum salt is in a range of 1 mmol/L to 20 mmol/L, and the concentration of the weak reducing agent is in a range of 1 mmol/L to 20 mmol/L.

## Patentansprüche

1. Ein Mikroelektroden-Array mit einer Modifikationsschicht, die auf einer Oberfläche einer Mikroelektrode davon angeordnet ist, wobei die Modifikationsschicht eine Platin-Nanosäulen-Modifikationsschicht umfasst, die mittels Elektroabscheidung angeordnet ist, **dadurch gekennzeichnet, dass** eine Iridiumoxidschicht, die mittels Platin-Nanosäulen-Modifikationsschicht vorgesehen ist; auf einer Oberfläche der Platin-Nanosäulen-Modifikationsschicht angeordnet ist und wobei die Platin-Nanosäulen-Modifikationsschicht und die Iridiumoxidschicht eine Iridiumoxid/Platin-Nanosäulen-Verbundbeschichtung bilden und wobei die Iridiumoxidschicht eine Dicke von 10 nm bis 500 nm aufweist.

2. Das Mikroelektroden-Array nach Anspruch 1, wobei die Platin-Nanosäulen-Modifikationsschicht eine Dicke von 500 nm bis 5 um hat und die Platin-Nanosäule der Platin-Nanosäulen-Modifikationsschicht einen Durchmesser von 50 nm bis 500 nm hat.

3. Das Mikroelektroden-Array nach Anspruch 1, wobei die Iridiumoxid/Platin-Nanosäulen-Verbundbeschichtung eine dreidimensionale nanoporöse Struktur aufweist und die poröse Iridiumoxid/Platin-Nanosäulen-Verbundbeschichtung einen Porendurchmesser von 50 nm bis 500 nm hat.

4. Ein Verfahren zur Herstellung einer Mikroelektrodenanordnung, bei dem eine Modifikationsschicht auf einer Oberfläche einer zu modifizierenden Mikroelektrode durch Elektroabscheidung von der Mikroelektrode hergestellt wird, wobei die Modifikationsschicht eine Platin-Nanosäulen-Modifikationsschicht umfasst, **dadurch gekennzeichnet, dass** das Verfahren umfasst:
(1) Zugabe einer geeigneten Menge eines schwachen Reduktionsmittels zu einer Platinsalzlösung unter Mischen, um eine Elektroabscheidungslösung A zu erhalten;
(2) Bilden eines ersten Drei-Elektroden-Systems unter Verwendung einer Platinplatte als Gegenelektrode, Ag/AgCI als Referenzelektrode, der zu modifizierenden Mikroelektrode als Arbeitselektrode" und der Elektroabscheidungslösung A, wobei das erste Drei-Elektroden-System mit einer elektrochemischen Arbeitsstation verbunden wird;
(3) Durchführen einer Elektroabscheidung bei normaler Temperatur und normalem Druck für 300 bis 600s so dass sich eine Platin-Nanosäulen-Modifikationsschicht auf der Oberfläche der Mikroelektrode bildet, um die Emikroelektrodenanordnung zu erhalten, wobei eine Iridiumoxidschicht auf der Oberfläche der Platin-Nanosäulen-Modifikationsschicht hergestellt wird,wobei das Verfahren ferner umfasst:
Zugabe einer geeigneten Menge eines Oxidationsmittels und einer schwachen Säure zu einer Iridiumsalzlösung unter Mischen, um eine Elektroabscheidungslösung B zu erhalten; Bilden eines zweiten Drei-Elektroden-Systems unter Verwendung einer Platinplatte als Gegenelektrode, Ag/AgCI als Referenzelektrode, der mit der Platin-Nanosäulen-Modifikationsschicht versehenen Mikroelektrodenanordnung als Arbeitselektrode und der Elektroabscheidungslösung B, das zweite Drei-Elektroden-System, das mit einer elektrochemischen Arbeitsstation verbunden ist und eine galvanische Abscheidung bei normaler Temperatur und normalem Druck für 300-600s durchführt, so dass sich die Iridiumoxidschicht mit einer Dicke von 10 nm bis 500 nm auf der Oberfläche der Platin-Nanosäulen-Modifikationsschicht bildet und eine Kombination aus der Platin-Nanosäulen-Modifikationsschicht und der Iridiumoxidschicht eine Iridiumoxid/Platin-Nanosäulen-Verbundbeschichtung bildet.

5. Das Verfahren zur Herstellung eines Mikroelektroden-Arrays nach dem Anspruch 4, wobei in der Elektroabscheidungslösung B die Konzentration des Iridiumsalzes in einem Bereich von 1 mmol/L bis 5 mmol/L, die Konzentration des Oxidationsmittels in einem Bereich von 1 mmol/L bis 5 mmol/L und die Konzentration der schwachen Säure in einem Bereich von 1 mmol/L bis 5 mmol/L liegt.

6. Das Verfahren zur Herstellung eines Mikroelektroden-Arrays nach dem Anspruch 4, wobei die Platinsalzlösung eine oder mehrere aus der Gruppe bestehend aus Platinnitrat, Platinchlorid, Chlorplatinsäure, Ammoniumhexachloroplatinat, Natriumchloroplatinat, Kaliumhexachloroplatinat und Kaliumtetrachloroplatinat ist.

7. Das Verfahren zur Herstellung eines Mikroelektroden-Arrays nach dem Anspruch 4, wobei das schwache Reduktionsmittel eines oder mehrere ist, ausgewählt aus der Gruppe bestehend aus Ameisensäure, Hydroxylammoniumchlorid, Zitronensäure, Citrat, Ascorbinsäure, Ascorbat, Benzol-1,4-diol, Benzol-1,2,3-triol und Benzol-1,2,4-triol.

8. Das Verfahren zur Herstellung eines Mikroelektroden-Arrays nach dem Anspruch 4, wobei in der Elektroabscheidungslösung A die Konzentration des Platinsalzes in einem Bereich von 1 mmol/L bis 20 mmol/L liegt und die Konzentration des schwachen Reduktionsmittels in einem Bereich von 1 mmol/L bis 20 mmol/L liegt.

## Revendications

1. Un réseau de microélectrodes comportant une couche de modification disposée sur une surface d'une microélectrode, dans laquel la couche de modification comprend une couche de modification de nanopiliers de platine disposée par électrodéposition, **caractérisée en ce qu'**une couche d'oxyde d'iridium éliminée par électrodéposition est fournie sur une surface de la couche de modification du nanopilier de platine; où la couche de modification des nanopiliers de platine et la couche d'oxyde d'iridium forment un revêtement composite d'oxyde d'iridium/nanopilier de platine et où la couche d'oxyde d'iridium a une épaisseur de 10 nm à 500 nm.

2. Le réseau de microélectrodes selon la revendication 1, dans lequel la couche de modification du nanopilier de platine a une épaisseur de 500 nm à 5 um, et le nanopilier de platine de la couche de modification du nanopilier de platine a un diamètre de 50 nm à 500 nm.

3. Le réseau de microélectrodes selon la revendication 1, dans lequel le revêtement composite d'oxyde iridium/nanopilier de platine a une structure nanoporeuse tridimensionnelle, et le revêtement composite poreux d'oxyde iridium/nanopilier de platine a un diamètre de pore de 50 um à 500um.

4. Méthode de préparation d'un réseau de microélectrodes, dans laquelle une couche de modification est préparée sur une surface d'une microélectrode pour être modifiée par électrodéposition à partir du réseau de microélectrodes, dans laquelle la couche de modification comprend une couche de modification de nanopilier de platine, **caractérisée en ce que** la méthode comprend:
(1) Ajouter une quantité appropriée d'un agent réducteur faible à une solution de sel de platine en mélangeant pour obtenir une solution d'électrodéposition A;
(2) Formation d'un premier système à trois électrodes utilisant une plaque de platine comme contre-électrode, Ag/AgCl comme électrode de référence, la microélectrode à modifier comme électrode de travail et la solution d'électrodéposition A, le premier système à trois électrodes raccordé à un poste de travail électrochimique;
(3) Effectuer l'électrodéposition à température et pression normales pour 300-600s de sorte qu'une couche de modification de nanopilier de platine se forme à la surface de la microélectrode pour obtenir le réseau de microélectrodes, où une couche d'oxyde d'iriduim est préparée sur une surface de la couche de modification de nanopilier de platine, la méthode comprend:
Ajouter une quantité appropriée d'agent oxydant et d'acide faible à une solution de sel iridium en mélangeant pour obtenir une solution d'électrodéposition B;
Formation d'un deuxième système à trois électrodes utilisant une plaque de platine comme contre-électrode Ag/AgCl comme électrode de référence, le réseau de microélectrodes fourni avec la couche de modification nanopilier de platine comme électrode de travail, et la solution d'électrodéposition B, le système relié à un poste de travail électrochimique; et effectuer l'électrodéposition à température et pression normales pour 300-600s de telle sorte que la couche d'oxyde iridium se forme à la surface de la couche de modification nanopaillar de platine et qu'une combinaison de la couche de modification nanopilier de platine et de la couche d'oxyde iridium forme un nanopilier d'oxyde iridium/platine revêtement composite.

5. La méthode de préparation d'un réseau de microélectrodes selon la revendication 4, dans laquelle dans la solution de microélectrodéposition B, la concentration du sel iridium est comprise entre 1 mmol/L et 5 mmol/L, la concentration de l'agent oxydant est comprise entre 1 mmol/L et 5 mmol/L, et la concentration de l'acide faible est comprise entre 1 mmol/L et 5 mmol/L.

6. Méthode de préparation d'un réseau de microélectrodes selon la revendication 4, dans laquelle la solution de sel de platine est l'une des plus sélectionnées dans le groupe constitué de nitrate de platine, de chlorure de platine, d'acide chloroplatinique, d'hexachloroplatinate d'ammonium, de chloroplatinate de sodium, hexachloroplatinate de potassium et tétrachoroplatinate de potassium.

7. Méthode de préparation d'un réseau de microélectrodes selon la revendication 4, dans laquelle l'agent réducteur faible est l'un des plus sélectionnés dans le groupe constitué d'acide formique, de chlorure d'hydroxylammonium, d'acide citrique, de citrate, d'acide ascorbique, d'ascorbate, de benzène-1,4-diol, de benzène-1,2,3-triol et benzène-1,2,4-triol.

8. La méthode de préparation d'un réseau de microélectrodes selon la revendication 4, dans laquelle dans la solution d'électrodéposition A, la concentration du sel de platine est comprise entre 1 mmol/L et 20 mmol/L, et la concentration de l'agent réducteur faible est comprise entre 1 mmol/L et 20 mmol/L.
